# EUROPEAN PATENT APPLICATION

(11) **EP 4 163 303 A1**
(43) Date of publication of application: **12.04.2023**
(21) Application number: 21822305.5
(22) Date of filing: 26.05.2021
(51) Int. Cl.: C07K 19/00, C12N 15/30, A61K 39/008, A61P 37/02

(54) **SYNTHETIC MULTI-EPITOPE CHIMERA AS A VACCINE AND TREATMENT FOR LEISHMANIASIS IN MAMMALS**

(30) Priority: 08.06.2020 ES 202030547
(71) Applicant: Universidad Complutense De Madrid, 28040 Madrid (ES)
(72) Inventor: MARTÍNEZ RODRIGO, Abel, 28791 - Madrid Soto del Real (ES); MAS ZUBIRI, Alicia, 28050 - Madrid Madrid (ES); CARRIÓN HERRERO, Francisco, Javier, 28770 - Madrid Colmenar Viejo (ES); ORDEN GUTIÉRREZ, José, Antonio, 28224 - Madrid Pozuelo de Alarcón (ES); DE LA FUENTE LÓPEZ, Ricardo, 28035 - Madrid Madrid (ES); DOMÍNGUEZ BERNAL, Gustavo, 28003 - Madrid Madrid (ES)
(74) Representative: Pons
(86) International application number: PCT/ES2021/070377
(87) International publication number: WO 2021/250299

(57) **Abstract**

Synthetic chimera which includes four multi-epitope peptides against Leishmania. Each of the peptides has been selected from a Leishmania infantum protein. These are nucleosomal histones H2A, H2B, H3 and H4. The invention also relates to a pharmaceutical composition which includes the synthetic chimera. It also concerns a prophylactic and/or therapeutic vaccine against Leishmania spp. for use in mammals and especially in humans and dogs.

## Description

### SECTOR OF THE ART

The present invention falls within the sector of human and animal health. More specifically, the invention relates to a synthetic multi-epitope chimera and its use in the preparation of a vaccine and an immunotherapeutic treatment against infection by *Leishmania spp.*

### BACKGROUND OF THE INVENTION

Leishmaniasis constitutes a group of diseases with worldwide distribution and caused by intracellular protozoan parasites of the genus *Leishmania,* which are transmitted by an insect vector known as sandfly. This disease exhibits several clinical and epidemiological patterns. According to data from the WHO, the prevalence of the disease is 12 million cases and it currently threatens 350 million people in 88 different countries, mainly in developing countries (Sudan, India, Bangladesh, Nepal and Brazil) located in tropical and subtropical areas, although it is also relevant in the Mediterranean basin.

Based on the clinical symptoms of the disease, leishmaniasis has been classified into several types. On the one hand, visceral leishmaniasis (VL) and, on the other, other leishmaniasis with cutaneous manifestations: cutaneous leishmaniasis (CL), diffuse cutaneous leishmaniasis (DCL) and mucocutaneous leishmaniasis (MCL). *Leishmania infantum* is the main causative agent of VL in the Mediterranean region where, furthermore, the domestic dog is considered the main reservoir, playing a key role in the onset of the disease in humans. Moreover, *L. major* is one of the species with the greatest impact on CL in Europe.

Despite its long history, leishmaniasis continues to be the fourth most common cause of death and disease, respectively, among tropical diseases. Furthermore, several studies on leishmaniasis have recently been published showing how the parasites are spreading to new geographical areas around the world by adapting to changing environments.

In this context, immunisation seems to be the best approach to control leishmaniasis in the long term, since current treatments are highly toxic and very expensive. Added to this is the possible development of resistance. Interestingly, while an infection by this parasite builds immunity, successful immunisation against *Leishmania spp.* will depend on the generation of specific memory T lymphocytes that can be maintained over time. An effective vaccine must also induce the development of CD8+ and CD4+ Th1 cellmediated antiparasitic immunity, which is characterised by the production of interferon-γ (IFN-γ) and IL-12, among other proinflammatory cytokines.

Despite extensive research to make progress towards the development of vaccines and better understand the immunopathogenesis of visceral leishmaniasis (VL), there is no approved vaccine for immunoprophylaxis or immunotherapy against human VL. There are, however, three vaccines available on the market for immunisation against canine leishmaniasis (LeishTec^{®}, CaniLeish^{®} and LetiFend^{®}) and, until 2014, there was another one that is now withdrawn (Leishmune^{®}).

WO2006122382A1 discloses the vaccine that was marketed in Brazil under the name Leishmune^{®} and that was subsequently withdrawn because its efficacy could not be demonstrated in phase III trials. It is a second generation vaccine, composed of the fucose-mannose ligand (FML) of *L. donovani* and saponin as an adjuvant.

LeishTec^{®} is the currently approved vaccine in Brazil for dogs. It comprises a *L. donovani* amastigote recombinant protein (A2 protein) and saponin as an adjuvant. There are several patents and patent applications that propose A2 as the protein of choice for the preparation of vaccines against *Leishmania:* WO2009089605A1, WO2002078735A2, US5733778, WO2019160971A1, among them.

CaniLeish^{®} has been marketed in Europe since 2011, composed of antigens secreted and excreted from *L. infantum* promastigotes, also with saponin as an adjuvant. ES2595207T3 discloses a therapeutic vaccine complex for the prevention or treatment of leishmaniasis, said complex composed of excretion and secretion molecules from *Leishmania spp.* amastigotes and/or promastigotes produced in a defined medium.

The fourth cited vaccine is LetiFend^{®}. It is a recombinant vaccine that contains a chimeric protein (Q protein) made up of five antigen fragments of four different *L. infantum* proteins (ribosomal proteins LiP2a, LiP2b and LiPO and histone H2A), described in ES2133236B1 and in ES2326174T3.

However, after several years on the market, doubts remain regarding the efficacy and effectiveness of these vaccines, the potential infectivity of vaccinated and infected animals, or the interference of vaccine-induced antibodies in the serological diagnosis of *L. infantum* (Velez, R.; Gallego, M. Commercially approved vaccines for canine leishmaniosis: a review of available data on their safety and efficacy. Trop Med Int Health. (2020) 25, 5:540-557.), such that the most widely used method to prevent the disease in dogs living in endemic areas continues to be the topical application of treatments with repellents and insecticides. However, even when used correctly, these products cannot fully protect dogs and further control measures must be adopted. In this regard, other possible vaccines seeking a solution to this problem have been described. Several examples are included below.

WO2014160987A2 discloses a fusion polypeptide comprising a non-specific nucleoside hydrolase (NH), a sterol 24-c-methyltransferase (SMT) and a polypeptide selected from the group consisting of HSP70, a cysteine polypeptidase B (CpB), a histone H2BN, a polypeptide A2 and a p21 antigen, or a eukaryotic initiation factor 4a (Leif), of *Leishmania spp.* as an immunostimulant.

WO2014102471A1 discloses a vaccine based on antigen/peptide fragments selected from the *Leishmania* soluble PSA (Promastigote Surface Antigen) virulence protein. WO2014091463A1 discloses a vaccine against *Leishmania* comprising at least one of the proteins HSP 83-1, MAPK and MAPK3.

WO2013011184A1 discloses a vaccine based on a chimeric molecule comprising the *L. infantum* PFR1 protein or a fragment of PFR1 with specific immunodominant epitopes, and which may also include HSP70.

ES2415516B2 relates to a multi-component chimera for use as a vaccine against infection by *Leishmania spp.* in mammals, including cutaneous leishmaniasis and visceral leishmaniasis, comprising 6 genes from *Leishmania infantum:* H2A, H2B, H3, H4, A2 and HSP70.

BR102014031331A2 proposes a recombinant immunogenic protein that contains immunogenic epitopes of several *Leishmania* proteins: tubulin alpha chain, heat shock proteins HSP83.1 and HSP70 and kinesin, for use in the production of vaccines, drugs and biopharmaceuticals to prevent and treat leishmaniasis.

It therefore considers the need for new solutions and new strategies to develop vaccines for human use or to prevent canine leishmaniasis.

### DESCRIPTION OF THE INVENTION

The invention relates to a synthetic multi-epitope chimera as a vaccine and treatment against leishmaniasis in mammals.

One aspect of the present invention relates to a synthetic multi-epitope chimera which includes multiple epitopes with four *Leishmania spp.* proteins previously described as immunogenic. More specifically, these epitopes are four nucleosomal histones: H2A, H2B, H3 and H4, which accession numbers in GenBank are: XP003392461, XP001263598, XP001463740 and XP001464339, respectively. A selection of epitopes has been made using algorithms from sequence analysis programs available on the Web, to choose epitopes with high binding affinity to major histocompatibility complex (MHC) class I and class II molecules from different mammal species (promiscuous epitopes).

MHC molecules are proteins that are encoded by more than 200 genes. The different loci that encode the MHC in each mammal species are highly polymorphic; in fact, they have the greatest intra-species genetic variability detected in Population Genetics. In other words, each specific locus that participates in the encoding of the MHC complex has a multitude of allelic variants within the natural populations of each species. The set of alleles that define a certain MHC combination in an individual is called a haplotype. In humans, these genes are found in different regions or loci depending on whether they encode MHC class I (loci HLA-A, HLA-B and HLA-C) or MHC class II (loci HLA-DR, HLA-DP and HLA-DQ). However, in mice they are found in the H-2 region with the loci (K, D and L) and (I-A and I-E)) for class I and II, respectively. The polymorphism of MHC molecules will determine differences in the capacity and affinity to bind to different epitopes.

In this specification, MHC-I is understood to be major histocompatibility complex class I; MHC-II is understood to be major histocompatibility complex class II. HLA is understood to be human leukocyte antigens (human MHC). H-2 is understood to be mice MHC.

The wide range of parasite epitopes that become visible to the host's immune system stimulates leishmanicidal immune responses in superior models (such as dogs, mice and humans), constituting an essential tool in the fight against different forms of leishmaniasis. After an *in silico* analysis of the sequences of the four histones mentioned above, not all the epitopes behave, *in vivo,* as predicted by the algorithms. Therefore, it is essential that the epitopes be correctly selected to obtain an adequate immune response to protect against leishmaniasis. To that end, the peptides obtained as a result of the *in silico* analysis of the sequences of the four selected proteins were analysed and modified to obtain four peptides that are able to generate a protective immune response against *Leishmania.*

A first analysis was based on two criteria: high affinity for MHC-I and maintained affinity for the largest possible number of alleles different from the loci that encode human and murine MHC-I. A second analysis was used to identify, for each of the four proteins, high-affinity epitopes for the alleles of the loci that encode human and murine MHC-II.

In this specification, affinity is understood to be the force of interaction between each of the epitopes with the different alleles of the problem loci, and high affinity is understood to be values of <0.5% for MHC-I and <2% for MHC-II, following the criteria established by the programs used in the bioinformatic analysis and which compare this affinity with a set of 400,000 random natural peptides.

The next step was a detailed study of the preselected sequences to identify those that bound with high affinity to the largest number of alleles of the loci that encode human and murine MHC class I molecules and that simultaneously encompass sequences with high binding affinity to a large number of alleles of the human and murine loci that encode MHC class II.

To end, the peptides selected up to that moment were modified by adding amino acids at their ends until 4 peptides with, at least, one high-affinity epitope with a large number of alleles of the loci that encode for human and murine MHC class I and II were obtained. The selected peptides, one for each protein, are shown in Table 1 and are characterised by SEQ ID NO: 1-4, with a length of 15-24 amino acids.

One aspect of the present invention relates to a synthetic multi-epitope chimera including, in a continuous sequence, the peptides characterised by SEQ ID NO: 1, SEQ ID NO: 2, SEQ ID NO: 3 and SEQ ID NO: 4 arranged in any order. Continuous sequence is understood to mean when the sequences of the four peptides are located one after the other without separation between them or with a maximum separation of two amino acids that allow the spatial configuration of the chimera to be improved for its processing and subsequent binding to the MHCs. The synthetic multi-epitope chimera may have, therefore, between 70 and 76 amino acids. It also relates to synthetic multi-epitope chimeras that exhibit 95% identity to any of the synthetic multi-epitope chimeras obtained by arranging in any order, and continuously, the peptides characterised by SEQ ID NO: 1, SEQ ID NO: 2, SEQ ID NO: 3 and SEQ ID NO: 4, giving rise to chimeras having 70-76 amino acids. Preferably, the synthetic multi-epitope chimera includes the peptides characterised by SEQ ID NO: 1, SEQ ID NO: 2, SEQ ID NO: 3 and SEQ ID NO: 4 in the order characterised by SEQ ID NO: 5, or in the same order but including up to 2 amino acids between peptides, or a sequence with 95% identity to SEQ ID NO: 5, or a sequence with 95% identity to the sequence that includes the peptides characterised by SEQ ID NO: 1, SEQ ID NO: 2, SEQ ID NO: 3 and SEQ ID NO: 4 in the same order and includes up to 2 amino acids between them.

In this specification, percentage identity of a sequence is understood to mean the percentage of matches of the same amino acids between two aligned sequences, along the entire length of both sequences.

These synthetic multi-epitope chimeras are able to protect animals against infection by *Leishmania,* as shown in the examination of the immune response generated in mice after immunisation with one of the synthetic multi-epitope chimeras with regard to the main biomarkers of protection, such as proinflammatory cytokines, the production of immunoglobulins specific to IgG1/IgG2 isotypes and the responses of CD4+ and CD8+ T cells.

Leishmaniasis occurs when there is an imbalance between an (inadequate) immune response and the parasite, which favours the appearance of clinical symptoms and/or the proliferation of the microorganism. The induction or modulation of an adequate immune response enables remission of the clinical pattern and enables the effectiveness of the treatment against the disease.

Therefore, another aspect of the invention relates to a pharmaceutical composition comprising a synthetic multi-epitope chimera among those described above, for use in mammals and, more specifically, for use in a prophylactic and/or therapeutic vaccine against *Leishmania spp.,* especially in humans and dogs. Furthermore, the pharmaceutical composition may include other elements such as pharmacologically acceptable excipients, or carriers or adjuvants that improve or enhance their effect. Among the adjuvants, saponin is preferably used.

Another aspect of the invention relates to a prophylactic and/or therapeutic vaccine against *Leishmania spp.* comprising a synthetic multi-epitope chimera or a pharmaceutical composition as described in the preceding paragraphs. This vaccine protects against leishmaniasis produced by *L. infantum,* which causes both VL and CL; therefore the synthetic multi-epitope chimeras described in this specification and the pharmaceutical compositions containing them are of interest for use in the preparation of a prophylactic and/or therapeutic vaccine in humans and/or dogs.

### BRIEF DESCRIPTION OF THE FIGURES

As a complement to the present description, and for the purpose of helping to make the features of the invention more readily understandable, said description is accompanied by a set of drawings constituting an integral part of the same, which by way of illustration and not limitation represent the following:

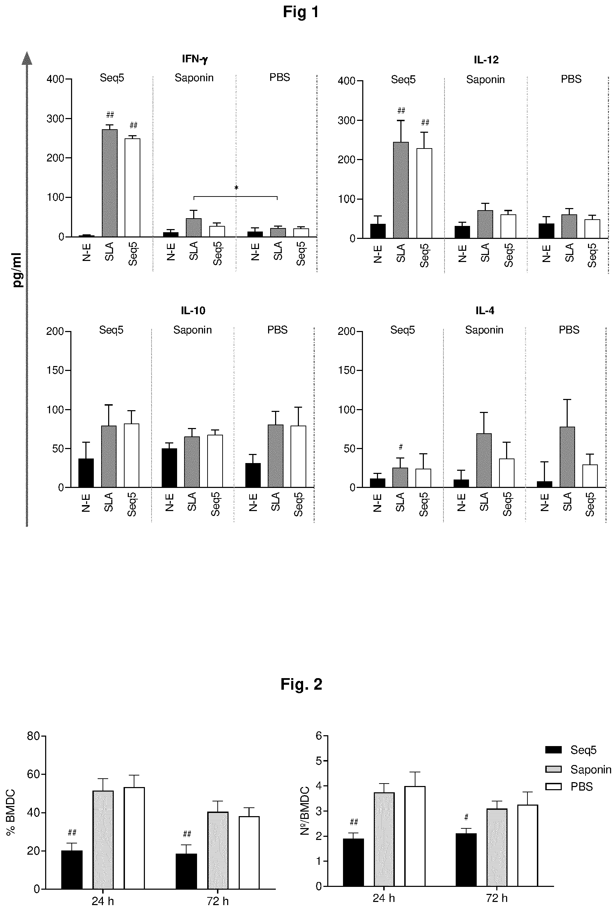

**Figure 1** shows the production (pg/ml) of specific proinflammatory cytokines (interferon-γ, IFN-γ; interleukin-12, IL-12) or anti-inflammatory cytokines (IL-10 and IL-4) in animals immunised with the synthetic multi-epitope chimera characterised by SEQ ID NO: 5 (Seq5) or inoculated with the Saponin or PBS controls and restimulated with the synthetic multi-epitope chimera characterised by SEQ ID NO: 5, with soluble *Leishmania* antigen (SLA) or with PBS.
**Figure 2** shows the percentage of infected target cells (% BMDC, bone marrow-derived dendritic cells) and the number of *Leishmania* amastigotes present per cell (No./BMDC) in co-cultures of virgin BMDCs infected with *L. infantum* and, subsequently, co-cultured with spleen cells from animals immunised with the synthetic multi-epitope chimera characterised by SEQ ID NO: 5 (Seq5), and from animals inoculated with Saponin or PBS controls.

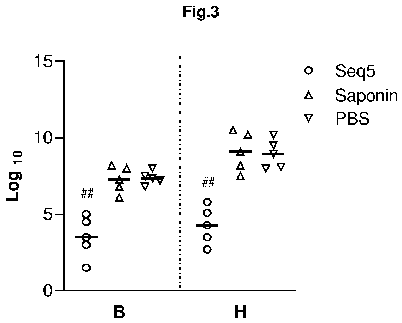

**Figure 3** shows the parasite load (Log₁₀) in animals immunised with the synthetic multi-epitope chimera characterised by SEQ ID NO: 5 (Seq5) and the animals inoculated with the Saponin and PBS controls and, subsequently, infected with *L. infantum,* both in the spleen (B) and the liver (H).

### PREFERRED EMBODIMENT OF THE INVENTION

The present invention is illustrated by means of the following examples that are not intended to limit the scope thereof.

### Example 1. Peptide selection.

To obtain the multi-epitope peptide chimera, firstly, multiple epitopes that are able to bind with high affinity to human and murine MHC-I and MHC-II molecules were selected. To do so, artificial neural networks were used to predict high-binding epitopes to human and murine MHC-I and MHC-II molecules and an *in silico* analysis was performed from four *Leishmania* antigens, the four histones: H2A, H2B, H3 and H4.

Since the human population has great genetic diversity in the loci that encode MHCs, we analysed the proteins to be studied with respect to all the allelic variations that encode the human MHCs available in the NetMHC program databases (version 4.0) (Andreatta M, Nielsen M Gapped sequence alignment using artificial neural networks: application to the MHC class I system. Bioinformatics (2016) 15;32(4):511-7; Nielsen M. et al. Reliable prediction of T-cell epitopes using neural networks with novel sequence representations. Protein Sci., (2003) 12:1007-17) and NetMHCll (version 2.3) (Jensen KK, et al. Improved methods for predicting peptide binding affinity to MHC class II molecules. Immunology (2018) 154(3):394-406) for MHC-I and MHC-II, respectively. The different human alleles of the loci *HLA* (A, B, D and E) were checked for the class I epitopes, and the human alleles of the loci *HLA* (DR, DP and DQ) were checked for the class II epitopes, in addition to the murine alleles of the loci *H-2* for class I molecules (K, D and L) and II (IA, I-E) of the MHC.

The analysis was carried out with each of the four proteins used following a similar protocol, which is summarised below. Firstly, an analysis was carried out on the entire sequence of each protein divided into epitopes with a size of 9 mer (amino acids) that meet two criteria: that they exhibit high affinity for MHC-I (understanding affinity as the force of interaction between each of the epitopes with the different alleles of the problem loci), and that this affinity is maintained for the largest possible number of alleles different from the loci that encode human and murine MHC-I. Next, a search was carried out, from the sequence of each protein, for 15-mer epitopes with high affinity for the alleles of the loci that encode human and murine MHC-II. Between 100 and 133 different and potentially useful peptides were obtained from each protein from the computer program.

Subsequently, a detailed study was conducted on the 15 amino acid sequences obtained. An analysis was carried out on which ones bound with high affinity to the largest number of alleles of the loci that encode human and murine MHC class I molecules, considering high affinity to be values <0.5%, following the criteria established by the programs used in the bioinformatic analysis and which compare this affinity with a set of 400,000 random natural peptides. Furthermore, it was established that said sequences had to encompass, simultaneously, 9 amino acid sequences that also exhibited high binding affinity to a large number of alleles of the human and murine loci that encode MHC class II (values <2% in this case). Lastly, some sequences were lengthened by manually adding amino acids to one or both ends, adding adjacent amino acids one by one to the preselected peptides of the protein in a variable number until each peptide had, at least, one high-affinity epitope with a large number of alleles (Table 2) of the loci where all the genes that encode human and murine MHC class I and class II are found.

**Table 1. Selected peptide of each histone and SEQ ID NO that characterises it.**

| Protein | Selected peptide | SEQ ID NO: |
|---|---|---|
| H2A | TAVLEYLTAELLELS | 1 |
| H2B | RSLKAINAQMSMHRTMKIVNSYV | 2 |
| H3 | EGLRFQSSAIMALQE | 3 |
| H4 | ITRGCVRRMARRGGVK | 4 |

**Table 2. Number of allelic variants of each loci with which each of the peptides shows high affinity.**

| | MHC-I | | | | MHC-II | | | |
|---|---|---|---|---|---|---|---|---|
| SEQ ID NO: | HLA-A | HLA-B | HLA-C and E | H-2 | HLA 17R | HLA-DP | HLA-DQ | H-2 |
| 1 | 13 | 6 | 8 | 1 | 5 | 9 | 15 | - |
| 2 | 16 | 12 | 9 | - | 16 | 2 | 11 | 4 |
| 3 | 10 | 3 | 8 | - | 17 | 5 | 12 | 3 |
| 4 | - | 6 | - | - | 6 | 1 | 3 | 2 |

As a result, we selected four peptides (one for each protein, as shown in Table 1), characterised by SEQ ID NO: 1-4 and with a length of 15-24 amino acids, having the largest number of high-affinity epitopes with restrictive binding to MHC-I (restricted being understood to mean essentially binding to MHC-I), as well as at least one adjacent or overlapping MHC-II epitope also restricted by its high score, according to the affinity values described above, and with high values in terms of their affinity for the largest number of human and murine alleles in the analyses of the bioinformatic programs. These characteristics are shown in Table 2.

### Example 2. Construction of a synthetic multi-epitope chimera.

Once the four multi-epitope peptides had been selected according to example 1, they were combined in a chimera according to the order: SEQ ID NO: 1 - 2 - 3 - 4, giving rise to the amino acid sequence TAVLEYLTAELLELSRSLKAINAQMSHRTMKIVNSYVEGL RFQSSAIMALQEITRGCVRRMARRGGVK, characterised by SEQ ID NO: 5, containing high-binding epitopes of the four histones H2A, H2B, H3 and H4.

Using the computer programs described in example 1, the chimera characterised by SEQ ID NO: 5 was analysed in depth, verifying that it conserved the MHC class I and II epitopes. Furthermore, it was analysed using a new software prediction of epitopes with affinity for binding to T lymphocytes and B lymphocytes (NetTepi, version 1.0) (Trolle T, Nielsen M. NetTepi: an integrated method for the prediction of T cell epitopes. Immunogenetics (2014). 66(7-8):449-56). The chimera characterised by SEQ ID NO: 5 was positive for 9 of the 13 different alleles of the human MHC (HLA) of the common supertypes HLA-A and B. Common supertypes are considered to be clusters of allelic variations with common structural and functional characteristics that enable them to recognise very similar peptide epitopes. This chimera exhibited, furthermore, a high-affinity 9-mer epitope for, at least, one allelic variant of the loci that encode the chimpanzee, macaque, cow and pig MHC-I molecules. Lastly, and to rule out possible autoimmune side effects, an analysis of the multi-epitope chimera was carried out using the BLAST tool (*Basic Local Alignment Search Tool*) to rule out the existence of an identity greater than 90% to the human and murine proteome.

### Example 3. Immunisation with the synthetic multi-epitope chimera characterised by SEQ ID NO: 5.

To assess the immunogenicity of the synthetic multi-epitope chimera characterised by SEQ ID NO: 5, inoculations were performed subcutaneously in three groups of mice (n=8/group). The animals of one group, referred to as Seq5, were inoculated with 50 µg of the synthetic chimera characterised by SEQ ID NO: 5 and 25 µg of saponin, as an adjuvant; the animals of another group, saponin control group, were inoculated with 25 µg of saponin; and the PBS control group was inoculated with PBS (buffer in which both the synthetic multi-epitope chimera characterised by SEQ ID NO: 5 and the adjuvant were transported). The inoculation was carried out on the left rear plantar pad on days 60, 45 and 30, with respect to day zero of infection, with a total volume of 50 µl.

### Example 4. Assessment of the immunogenicity of the synthetic multi-epitope chimera characterised by SEQ ID NO: 5.

Mice immunised with the chimera characterised by SEQ ID NO: 5, following the instructions of example 3, exhibited an immune response characterised by a protective Th1 cell response profile with the production of proinflammatory cytokines (IFN-y, IL-12) against the profile exhibited by the Saponin and PBS controls that had a predominantly Th2-type immune response, compatible with a susceptibility profile against infection by *Leishmania* and characterised by the production of anti-inflammatory cytokines (IL-4, IL-10). The predominance of the Th1-type response coincides with a redirection of the specific humoral response against the synthetic multi-epitope chimera to the IgG2a isotype related to a Th1 response. The displacement of the immune response in the group immunised with the chimera characterised by SEQ ID NO: 5 correlates with increased leishmanicidal activity in target cells infected with *Leishmania.*

Figure 1 shows the production of specific cytokines against restimulation with the chimera characterised by SEQ ID NO: 5 (indicated in the figure as Seq5) in animals immunised as described in example 3. Use was made of virgin bone marrow-derived dendritic cells (BMDC) stimulated with soluble *Leishmania* antigen (SLA; produced with the supernatants obtained after having lysed cultures of *Leishmania* promastigotes obtained after sonication and subsequent centrifugation), grey bars; or stimulated with the chimera characterised by SEQ ID NO: 5, white bars; unstimulated BMDC was used as negative control, indicated as N-E with black bars. The BMDCs were co-cultured with spleen cells, splenocytes, of the animals previously immunised as indicated in example 3. Cytokine production (pg/ml) was quantified using the ELISA technique. The data are presented as the mean ± standard deviation (SD). The hash marks indicate statistically significant differences (#, *P <* 0.05; ##, *P* <0.001) between the immunised groups and the two control groups (Saponin and PBS). The asterisk indicates statistically significant differences (*, *P* < 0.05; **, *P* < 0.001). Figure 1 shows how the splenocytes from the group immunised with the chimera characterised by SEQ ID NO: 5 produce a greater amount of IFN-γ, IL-12 and that this production is specific, in response to a previous stimulation with the chimera characterised by SEQ ID NO: 5 or with a set of total *Leishmania* antigens (SLA), but this does not occur when stimulating with PBS. Splenocytes from animals immunised with Saponin or PBS do not show this production of proinflammatory cytokines under any of the stimuli (SLA or chimera characterised by SEQ ID NO: 5).

Figure 2 shows the percentage of infected target cells (% BMDC) and the number of intracellular amastigotes detected per cell (No./BMDC) at 24 and 72 hours of postinfection incubation. The leishmanicidal activity of the chimera characterised by SEQ ID NO: 5 (indicated in the figure as Seq5) was assessed in BMDC. Virgin BMDCs infected with *L. infantum* were co-cultured with splenocytes from animals previously immunised with the chimera characterised by SEQ ID NO: 5 as indicated in example 3, and with splenocytes from animals previously inoculated with the two controls used according to example 5. The parameters of infected cells and number of intracellular amastigotes were determined by optical microscopy. The data are presented as the mean ± SD. The hash marks indicate statistically significant differences (#, P < 0.05; ##, P < 0.001) between the immunised groups (black bars) and the two control groups: Saponin (grey bars) and PBS (white bars). As shown in Figure 2, a significant increase is observed in the leishmanicidal potential that they induce in the BMDCs infected with *L. infantum* in the splenocytes from animals immunised with the chimera SEQ ID NO: 5 compared to the splenocytes of the animals in the control groups. The increase in leishmanicidal capacity is observed with a notable reduction in the percentage of infected BMDCs at 24 and 72 hours, as well as a lower number of intracellular amastigotes. This result correlates with the predominant protective immune response in the group immunised with SEQ ID NO: 5 as described above in this example and figure 1.

### Example 5. Protection against L. infantum in the murine model of visceral leishmaniasis.

30 days after the last immunisation carried out as described in example 3, an infection was intravenously performed with 5×10⁵ infective *L*. *infantum* promastigotes in the 3 groups of animals (which we have named Seq5, Saponin and PBS). 6 weeks after infection, the animals were euthanised and the parasite load in the VL target organs was assessed. The parasite count was carried out by decimal dilutions of spleen and liver macerates, separately, and their subsequent observation under a microscope. As shown Figure 3, it was observed that the animals immunised with the chimera characterised by SEQ ID NO: 5 (Seq5) exhibited a lower parasite load both in the spleen (B) and in the liver (H), compared to the Saponin and PBS groups. In other words, immunisation with SEQ NO: 5 is able to drastically decrease the amount of *Leishmania* that is present in the liver and spleen compared to the control groups.

Therefore, we conclude that the immune response induced during immunisation with the chimera characterised by SEQ ID NO: 5 is able to protect against infection with *Leishmania* and, more specifically, with *L. infantum.*

### SEQUENCE LIST FREE TEXT

### SEQ ID NO: 5

Artificial sequence
Multi-epitope chimera composed of SEQ ID NO: 1, 2, 3 and 4 of *Leishmania infantum*

## Claims

1. A synthetic multi-epitope chimera comprising:
a.- the peptides **characterised by** SEQ ID NO: 1, SEQ ID NO: 2, SEQ ID NO: 3 and SEQ ID NO: 4, arranged in any order and separated by 0-2 amino acids, giving rise to a sequence of 70-76 amino acids; or
b.- modifications of the peptides **characterised by** SEQ ID NO: 1, SEQ ID NO: 2, SEQ ID NO: 3 and SEQ ID NO: 4, arranged in any order and separated by 0-2 amino acids, giving rise to a sequence of 70-76 amino acids with, at least, 95% identity to the sequence defined in a.-.

2. The chimera according to claim 1 **characterised by** SEQ ID NO: 5 or with, at least, 95% identity to SEQ ID NO: 5.

3. A pharmaceutical composition comprising a synthetic multi-epitope chimera defined in any of claims 1-2, for use in mammals.

4. The pharmaceutical composition according to claim 3 comprising a pharmacologically acceptable excipient, carrier and/or adjuvant.

5. The pharmaceutical composition according to claim 4, wherein the adjuvant is saponin.

6. The pharmaceutical composition according to any of claims 3-5 for use in a prophylactic and/or therapeutic vaccine against *Leishmania spp.*

7. A prophylactic and/or therapeutic vaccine against *Leishmania spp.* comprising a synthetic multi-epitope chimera according to any of claims 1-2 or a pharmaceutical composition according to any of claims 3-6.

8. The prophylactic and/or therapeutic vaccine according to claim 7 for use against *Leishmania infantum.*

9. The prophylactic and/or therapeutic vaccine according to any of claims 7-8 for use in humans and/or dogs.

## Amended claims

### Amended claims under Art. 19.1 PCT

1. A synthetic multi-epitope chimera consisting of SEQ ID NO: 5.

2. A pharmaceutical composition comprising the synthetic multi-epitope chimera defined in claim 1, for use in mammals.

3. The pharmaceutical composition according to claim 2 comprising a pharmacologically acceptable excipient, carrier and/or adjuvant.

4. The pharmaceutical composition according to claim 3, wherein the adjuvant is saponin.

5. The pharmaceutical composition according to any of claims 2-4 for use in prophylactic and/or therapeutic vaccination against *Leishmania spp.*

6. A prophylactic and/or therapeutic vaccine against *Leishmania spp.* comprising the synthetic multi-epitope chimera according to claim 1 or the pharmaceutical composition according to any of claims 2-5.

7. The prophylactic and/or therapeutic vaccine according to claim 6 for use against *Leishmania infantum.*

8. The prophylactic and/or therapeutic vaccine according to any of claims 6-7 for use in humans and/or dogs.
